# EUROPEAN PATENT APPLICATION

(11) **EP 4 834 204 A1**
(43) Date of publication of application: **04.11.2026**
(21) Application number: 25173645.0
(22) Date of filing: 30.04.2025
(51) Int. Cl.: A61B 5/00, A61N 1/37, A61B 5/349, A61N 1/362

(54) **ACTIVE IMPLANTABLE MEDICAL DEVICE TO DETECT NARROWLY MISSED THERAPY INCIDENTS**

(71) Applicant: BIOTRONIK SE & Co. KG, 12359 Berlin (DE)
(72) Inventor: Fischer, René, 10245 Berlin (DE)
(74) Representative: Biotronik Corporate Services SE

(57) **Abstract**

Active implantable medical device (10) for stimulating a human or animal heart that features the following steps during operation: a) detecting, with at least one detecting unit (13), a cardiac signal comprising a plurality of cardiac rhythm intervals; b) determining a length of each individual cardiac rhythm interval; c) incrementing a first counter (1) subsequent to meeting a start condition, if the length of an individual cardiac rhythm interval is below a predeterminable first threshold; d) releasing a stimulation for the human or animal heart by at least one stimulation unit (14), when the first counter (1) meets a first condition; and e) determining a maximum value of a degree of fulfillment of the first condition by the first counter (1) within a first time period since the start condition when a second condition is met; f) storing the maximum value (3) in at least one memory unit (12)..

## Description

The present invention relates to an active implantable medical device according to the preamble of claim 1, to a method for storing information detected by such an active implantable medical device according to the preamble of claim 14, and to a computer program product according to the preamble of claim 15.

Active implantable medical devices are used for many applications in stimulating a human or animal heart. These active implantable medical devices should apply a stimulation only in such cases in which the stimulation is indeed needed. However, due to an erroneous detection, the active implantable medical device might inadvertently cause stimulations that are not needed and therefore are considered inadequate. Such an erroneous detection might be caused by a loss of integrity of a detecting unit of the active implantable medical device, by environmental influences like magnetic fields and/or radio waves that impact the functionality of the active implantable medical device, and/or by parameter settings of the active implantable medical device that are not optimized to a patient carrying the active implantable medical device. Preventing such inadequate stimulations is an important task as inadequate stimulations can be painful or even harmful to the patient.

An inadequate stimulation in known active implantable medical devices is prevented by a medical practitioner manually adjusting parameter settings of the active implantable medical device via an external device, if it can be determined that the inadequate stimulations are most likely caused by unfavorable parameter settings. For doing so, the medical practitioner is evaluating inadequate stimulations that already occurred. In this case, the medical practitioner is relying on past occurrences of inadequate stimulations to prevent future inadequate stimulations.

Additionally or alternatively, the medical practitioner can evaluate a medical history of the patient. The medical history of the patient can include hints that the patient might be at risk of receiving inadequate stimulations from the active implantable medical device the patient carries.

Additionally or alternatively, the medical practitioner can assess the risk of the patient being subjected to inadequate stimulations with an evaluation of non-sustained (ventricular) tachycardia (NS(V)T) episodes detected by the active implantable medical device. NS(V)T episodes are good indicators for an underlying medical condition of the patient that might lead to inadequate stimulations but also for non-medical factors that can cause inadequate stimulations. The evaluation of the NS(V)T episodes is typically performed by the medical practitioner looking at data received from the active implantable medical device. However, NS(V)T episodes are quite common and storing capacities as well as transmission capacities of known active implantable medical devices are limited. Therefore, storing or transmitting all NS(V)T episodes is for most patients not feasible and even if it were feasible, the amount of data that would need to be evaluated extends an amount the medical practitioner could realistically evaluate in its entirety. Thus, the medical practitioner has typically only access to a sample of all NS(V)T episodes, and even if the medical practitioner had access to all NS(V)T episodes, the medical practitioner could only evaluate a sample. For this reason, this evaluation is not reliable.

Therefore, it is crucial to gain a better understanding of data collected by the active implantable medical device comprising detected signals from the human or animal heart that do not lead to a stimulation. A better understanding can help in preventing inadequate stimulations by guiding the medical practitioner in their evaluation of the risk of the patient being subjected to inadequate stimulations. If the data is better understood, a medical condition of the patient that increases the risk being subjected to inadequate stimulations might be noticeable without having to rely on past occurrences of inadequate stimulations, the medical history of the patient or an unreliable evaluation of NS(V)T episodes. Additionally, if the data is better understood, the evaluation of NS(V)T episodes can be guided by determining crucial or critical NS(V)T episodes which the medical practitioner then can evaluate in detail. Also, determining crucial or critical NS(V)T episodes reliably makes it possible to automatize the evaluation. An automatized evaluation could lead to an active implantable medical device that can determine more reliably, if a stimulation is necessary or not. In such an active implantable medical device, parameter setting adjustments might be more specific for and effective in preventing inadequate stimulations.

Adjusting the parameter settings of the active implantable medical device typically includes adapting the criteria to determine if by the active implantable medical device detected cardiac signal should induce a stimulation. However, such adjustments cannot completely prevent inadequate stimulations caused by the non-medical factors like environmental influences or a loss of integrity of electrodes of the active implantable medical device. Such adjustments might even make it more difficult to notice the non-medical factors early. For example, if the integrity of electrodes of the active implantable medical device is corrupted, then this loss of integrity is most likely only noticed after an oversensing scenario, followed by inadequate stimulation. But because the parameter settings were adjusted, the loss of integrity needs to be more severe to cause the inadequate stimulation.

Therefore, it would be desirable to gain a better understanding of the data collected by the active implantable medical device comprising detected signals from the human or animal heart that do not lead to a stimulation, to notice the non-medical factors that can impact a detection of the active implantable medical device. If such data is better understood, the non-medical factors impacting the detection of the active implantable medical device can be noticed more easily. For example, if the data comprises hints for a loss of integrity of the electrodes of the active implantable medical device, this information can be gained without an inadequate stimulation having to occur first. Moreover, as soon as the non-medical factor impacting the detection of the active implantable medical device can be noticed, preventive or counter measurements and/or actions can be initiated.

It is an object of the present invention to provide an implantable medical device that can improve the understanding of the data collected by the active implantable medical device comprising detected signals from the human or animal heart that do not lead to a stimulation.

This object is achieved with an implantable medical device having the claim elements of claim 1. Such an active implantable medical device serves for stimulating a human or animal heart. The active implantable medical device comprises a processor, at least one memory unit, at least one detecting unit configured to detect signals from the human or animal heart, and at least one stimulation unit configured to stimulate the human or animal heart.

According to an aspect of the present invention, the memory unit comprises a computer-readable program that causes the processor to perform the steps explained in the following when being executed on the processor.

In one step, a cardiac signal is detected, in particular continuously or repeatedly detected, with the at least one detecting unit. The cardiac signal usually comprises a plurality of cardiac events and cardiac rhythm intervals, wherein two consecutive cardiac events in each case limit a cardiac rhythm interval, wherein a plurality of consecutive cardiac rhythm intervals is generally indicative for a cardiac rhythm of a human or animal heart. The plurality of cardiac events can for example be a plurality of QRS complexes and the plurality of cardiac rhythm intervals can for example be a plurality of RR intervals. An RR interval thereby describes an interval between the two R waves of two consecutive QRS complexes. From measurements of the RR intervals a heart rate variability of the human or animal heart can be derived. Moreover, a cardiac frequency of the human or animal heart measured in beats per minute (bpm) can be determined from the RR intervals for example by dividing one minute by a length of one RR interval of the RR intervals converted in minutes.

In another step, a length of all individual cardiac rhythm intervals of the plurality of consecutive cardiac rhythm intervals is determined.

In yet another step, a first counter is incremented, in particular by a predeterminable first increment, if the length of an individual cardiac rhythm interval of the plurality of cardiac rhythm intervals is below a predeterminable first threshold. The incrementing of the first counter is only initiated upon meeting of a start condition. The first counter can be used for example as a measurement to determine if the human or animal heart is experiencing a ventricular tachycardia (VT) episode or a ventricular fibrillation (VF) episode. If the individual cardiac rhythm interval of the plurality of cardiac rhythm intervals is below the predeterminable first threshold, this indicates a faster cardiac rhythm, which might be indicative for example for VT or VF episodes.

In an embodiment the start condition may be fulfilled, when the length of each of at least two consecutive cardiac rhythm intervals is below the predeterminable first threshold. In another embodiment, the start condition may be a start time of a constant time period.

The predeterminable first threshold lies, in an embodiment, in a range of 250 ms to 750 ms, in particular 270 ms to 600 ms or in particular in a range of 330 ms to 660 ms, in particular in a range of 375 ms to 600 ms, in particular in a range of 430 ms to 545 ms, in particular in a range of 500 ms to 545 ms, or in particular 300 ms to 400 ms or 240 ms to 400 ms or 260 ms to 300 ms. In case the cardiac rhythm interval is the RR interval, these ranges correspond approximately to cardiac frequencies lying within a range of 80 bpm to 250 bpm, in particular within a range of 90 bpm to 180 bpm, in particular within a range of 100 bpm to 160 bpm, in particular within a range of 110 bpm to 140 bpm, in particular within a range of 110 bpm to 120 bpm.

The predeterminable first increment lies, in an embodiment, in a range of 1 to 10, in particular 2 to 7, in particular 3 to 6, in particular 4 to 5. The predeterminable first increment can in particular amount to 1.

In yet another step, a stimulation for the human or animal heart by the at least one stimulation unit is released, when, in particular immediately when the first counter meets a first condition. Moreover, a maximum value of a degree of fulfillment of the first condition by the first counter within a first time period since the start condition is determined, when a second condition is met.

In an embodiment, the first time period comprises a number of consecutive cardiac rhythm intervals which number may lie within a range of 10 to 60, in particular within a range of 12 to 50, in particular within a range of 15 to 40, in particular within a range of 20 to 35, in particular within a range of 25 to 30.In an embodiment, the first condition is a ventricular tachycardia and/or ventricular fibrillation detection criterion.

In an embodiment, the first time period is a time interval that is started upon fulfillment of the start condition and ended upon fulfillment of the second condition. The first time period may have a predeterminable constant length or may depend on changes in the cardiac rhythm.

In an embodiment, the second condition may be a supraventricular tachycardia detection criterion or a predeterminable end time of the first time period.

If the first counter meets the first condition, then it can be assumed that the human or animal heart is in need of a stimulation. This need could be due to a detection of VT and/or VF episodes. If VT and/or VF episodes are supposed to be detected then the first condition can differ depending on whether a VT or VF episode is supposed to be detected, but the first condition can also be the same for VT and VF episodes.

The first condition can be a predeterminable stimulating threshold lying, in an embodiment, in a range of 10 to 100, in particular 24 to 60 or 24 to 40.

In an optional method step, the first counter is reset to zero or decremented by a predeterminable number such as the first increment when a stimulation for the human or animal heart by the at least one stimulation unit has been released. In another embodiment, the first counter is reset to zero if a majority of all individual cardiac rhythm intervals of the plurality of consecutive cardiac rhythm intervals detected within the the first time period comprises a length that is equal to or exceeds the predeterminable first threshold.

In an example, the predeterminable first increment is 1 and the predeterminable stimulating threshold equals the number of consecutive cardiac rhythm intervals in the first time period. Thus, all cardiac rhythm intervals within the first time period are below the predeterminable first threshold, when releasing the stimulation.

In another example, the predeterminable first increment is 1 and the predeterminable stimulating threshold is smaller than the number of consecutive cardiac rhythm intervals. Thus, not all cardiac rhythm intervals within the first time period need to be below the predeterminable first threshold to release the stimulation. A typical value for the predeterminable stimulating threshold for such an example amounts to 18 and a typical value for such an example for the number of consecutive cardiac rhythm intervals amounts to 24. Another typical value for the predeterminable stimulating threshold for such an example amounts to 30 and a typical value for such an example for the number of consecutive cardiac rhythm intervals amounts to 40. This example is typical for detections of VF episodes.

If the second condition is met, then there is no need for a stimulation of the human or animal heart. A quantitative value can be determined to measure to which degree the first condition was reached. For instance, if the first condition is to reach a predeterminable stimulating threshold, the first increment amounts to 1, the first counter assumes a value of 27, and the predeterminable stimulating threshold is set to 30, then the quantitative value could be the percentage of the first counter compared to the predeterminable stimulating threshold, which would be 90 % in this example. However, other metrics to measure the degree of fulfillment of the predeterminable stimulating threshold are also possible.

In yet another step, a maximum value for a degree of fulfillment of the first condition by the first counter (1) within the first time period since the start condition is determined. By determining the maximum value for the first time period, the maximum value can be seen as representative for the first time period. If the maximum value is small, it can be concluded that there was not a narrowly missed therapy incident within the first time period, therefore simplifying an identification of narrowly missed therapy incidents by making it easy to determine irrelevant or less relevant missed therapy incidents.

In an embodiment, if the first time period is a predeterminable constant time period, the first time period may be 24 h for obtaining daily information and for daily information transmission. In another embodiment, the predeterminable first time period lies in a range of 1 hour to 24 hours, in particular in a range of 2 hours to 23 hours, in particular in a range of 3 hours to 22 hours, in particular in a range of 4 hours to 21 hours, in particular in a range of 5 hours to 20 hours, in particular in a range of 6 hours to 19 hours, in particular in a range of 7 hours to 18 hours, in particular in a range of 8 hours to 17 hours, in particular in a range of 9 hours to 16 hours, in particular in a range of 10 hours to 15 hours, in particular in a range of 11 hours to 14 hours, in particular in a range of 12 hours to 13 hours. The first time period may be selected to last 4 h to 6 h to provide 6 to 4 measurement intervals per day to probably detect daily routines. The first time period may also lie in a range of 1 to 30 days to provide longer intervals, e.g. for "offline" devices which have only every X months an in-office follow up in clinic.

In yet another step, maximum value is stored in the at least one memory unit. By storing maximum value, the maximum value can be evaluated at a later point. In another embodiment, also multiple local maximum values might be stored. This might be achieved by not only determining the maximum value in the previous step but determining also other local maximum values that have been determined within the first time period and storing all of them or, e.g., 5 to 100, such as 5, 10, 20, 25, 50 or 100 local maximum values.

Summarizing, the active implantable medical device allows a better understanding of the data comprising detected signals from the human or animal heart that have not led to a stimulation by identifying narrowly missed therapy incidents. Narrowly missed therapy incidents are for example incidents in which the criteria to induce a stimulation were almost (but not fully) fulfilled, and therefore a therapy in form of a stimulation has been missed by a narrow margin. Thus, narrowly missed therapy incidents indicate how close the active implantable medical device has been to releasing a stimulation.

In an embodiment, the computer-readable program also causes the processor to additionally perform the steps explained in the following when being executed on the processor.

In one step, it is evaluated if the maximum value is fulfilling a predeterminable criticality criterion. The predeterminable critical criterion serves for evaluating the maximum value. If the maximum value does not fulfill the predeterminable criticality criterion, the maximum value is determined to have no or only a low significance, i.e., to be non-critical, implying that the active implantable medical device was not close to initiating a stimulation. If the maximum value does fulfill the predeterminable criticality criterion, the maximum value is determined to be critical and therefore can be considered as representing a narrowly missed therapy incident.

In another step, a signal generation is triggered and/or a criticality counter is incremented by a criticality increment and a value of the criticality counter assumed by the criticality counter at the end of a predeterminable second time period is stored in the at least one memory unit, if the predeterminable criticality criterion is fulfilled. The predeterminable second time period comprises at least the first time period.

By triggering a signal generation, a response to the maximum value is possible. For instance, the signal generation might comprise setting a flag within the active implantable medical device so that, when an external device is connected to the active implantable medical device, the flag might show up as a warning on the external device. The connection to the external device might thereby be a wireless connection. The external device might be useable by a medical practitioner who can see the warning and investigate further and/or initiate an action to resolve an issue the warning might indicate.

The purpose of the criticality counter is to measure the criticality of every maximum value within the predeterminable second time period and measuring an overall criticality for the predeterminable second time period. This might simplify an evaluation as for example the medical practitioner might only have to evaluate a most critical time period of multiple predeterminable second time periods. Moreover, the criticality counter might make it possible to recognize trends like a sudden increase of criticality for multiple consecutive predeterminable second time periods.

The criticality of one maximum value is measured by the criticality increment, thus, the criticality increment might differ for every maximum value within the predeterminable second time period. The criticality increment might equal the maximum value itself as a measurement of how critical the maximum value is. However, the criticality increment can also weight other factors. In one example, the criticality increment is 1, in that case the overall criticality for the predeterminable second time period amounts to the amount of maximum values within the predeterminable second time period that are considered critical.

Thus, the predeterminable criticality criterion serves for determining whether the maximum value is critical, while the purpose of the criticality increment is to measure how critical the maximum value is.

The predeterminable second time period may be 24 h or may lie in a range of 12 hours to 1 year, in particular 12 hours to 10 days, in particular in a range of 24 hours to 8 days, in particular in a range of 36 hours to 7 days, in particular in a range of 48 hours to 6 days, in particular in a range of 60 hours to 5 days, in particular in a range of 72 hours to 4 days. Moreover, the predeterminable second time period may lie in a range of 7 to 30 days.

The predeterminable second time period can be as long as the first time period; in that case, the criticality counter reflects only the criticality of one maximum value.

In an embodiment, the predeterminable criticality criterion is fulfilled, if the maximum value is equal to or exceeds a predeterminable first degree of fulfillment of the first condition.

The predeterminable first degree of fulfillment of the first condition determines a degree of fulfillment of the first condition that is so high that if the maximum value is equal to it or exceeds it, the maximum value should be considered critical. Typically, the predeterminable first degree of fulfillment of the first conditon will amount to a comparatively large value because the criticality criterion is already fulfilled, if the maximum value is equal to or exceeds the predeterminable first degree of fulfillment of the first condition.

Additionally or alternatively, the predeterminable criticality criterion is fulfilled, if the maximum value is equal to or exceeds a predeterminable second degree of fulfillment of the first condition, wherein the predeterminable second degree of fulfillment of the first condition is smaller than the predeterminable first degree of fulfillment of the first condition, and if, at the same time, a majority of cardiac events detected within the first time period fulfills a first morphological criterion.

The predeterminable second degree of fulfillment of the first condition has a smaller value than the predeterminable first degree of fulfillment of the first condition. Therefore, a second condition needs to be fulfilled to fulfill the criticality criterion. This second condition comprises an evaluation of the morphology of multiple cardiac events associated with the maximum value. Such an evaluation is based on the fulfillment of the first morphological criterion. The morphological criterion can be based on an assessment of anomalies of the cardiac events. The morphological criterion might be fulfilled, if the cardiac event is classified as conspicuous or unhealthy. The cardiac event might be classified as conspicuous, if it deviates more than a standard deviation from a typical or healthy cardiac event. In one example, the morphological criterion evaluates QRS complexes.

Additionally or alternatively, the predeterminable criticality criterion is fulfilled, if the maximum value is equal to or exceeds a predeterminable absolute threshold. The predeterminable absolute threshold is independent of the first condition, while still being smaller than a threshold for meeting the first condition. With the predeterminable absolute threshold, all maximum values equal to or exceeding the predeterminable absolute threshold are considered critical. By setting the predeterminable absolute threshold, the medical practitioner has for example a simple method to control which maximum values are seen as critical. Therefore, the medical practitioner can easily influence which maximum values will be classified as critical. Such critical values can be analyzed by the medical practitioner or by an automatic evaluation at a later time point upon evaluating the data.

In an embodiment, the predeterminable first degree of fulfillment of the first condition lies within a range of 70 % to 95 %, in particular within a range of 80 % to 93 %, in particular within a range of 84 % to 91 %. The predeterminable first degree of fulfillment of the first condition amounts in particular to 90 %.

In an embodiment, the predeterminable second degree of fulfillment of the first condition lies within a range of 60 % to 90 %, in particular within a range of 64 % to 81%, in particular within a range of 69 % to 76 %. The predeterminable second degree of fulfillment of the first condition amounts in particular to 70 %.

In an embodiment, the predeterminable absolute value lies in a range of 6 to 95, in particular 14 to 36, or 10 to 29, in particular in a range of 15 to 27, in particular in a range of 20 to 27, in particular in a range of 20 to 25.

In an embodiment, the computer-readable program causes the processor to determine local maximum values of a degree of fulfillment of the first condition by the first counter within the first time period and to store the local maximum values in the memory unit, wherein the predeterminable criticality criterion is fulfilled, if a predeterminable number of the local maximum values and the maximum value of a degree of fulfillment of the first condition within the first time period is equal to or exceeds a predeterminable third degree of fulfillment of the first condition. Local maximum values are defined as local peak counter values after an increase of the counter value and before a decrease of the counter value. If there is a temporal accumulation of local maximum values that are equal to or exceed the predeterminable third degree of fulfillment of the first condition, then the maximum value fulfills the criticality criterion. These local maximum values might be below the predeterminable second degree of fulfillment of the first condition, but due to the temporal accumulation of these local maximum values they might indicate a medical condition and therefore should be considered critical.

The number of local maximum values typically can encompasses at least a fourth of all quantitative values within the first time period, in particular at least a third of all local maximum values within the first time period, in particular at least a half of all local maximum values within the first time period.

In an embodiment, the criticality increment is weighted with a weighting factor of higher than 1 if more than one condition leading to the fulfillment of the predeterminable criticality criterion is fulfilled.

In an embodiment, the predeterminable third degree of fulfillment of the predeterminable stimulating threshold lies within a range of 40 % to 60 %, in particular within a range of 45 % to 55 %, in particular within a range of 47 % to 53 %, in particular within a range of 49 % to 51 %. The predeterminable third degree of fulfillment of the first condition amounts in particular to 50 %.

In an embodiment, the criticality increment is weighted with a weighting factor of higher than 1 if the cardiac events detected within the first time period fulfills a second morphological criterion. The second morphological criterion evaluates at least one other morphological aspect of the cardiac events than the first morphological criterion. The second morphological criterion might be fulfilled, if the cardiac events comprise components that are not part of a QRS complex and/or if signal components of the cardiac events comprise a high frequence.

In an embodiment, the computer-readable program causes the processor to additionally perform the following steps:
In one step, a second counter is incremented, in particular by a predeterminable second increment, subsequent o meeting the start condition, if the length of an individual cardiac rhythm interval of the plurality of cardiac rhythm intervals is below the predeterminable first threshold. This step is performed preferably at the same time as incrementing the first counter or immediately before or immediately after incrementing the first counter.

In an embodiment, the second condition is met, when the second counter meets a third condition, wherein the third condition is a supraventricular detection criterion.

The predeterminable second increment lies, in an embodiment, within a range of 1 to 10, in particular within a range of 2 to 9, in particular within a range of 3 to 8, in particular within a range of 4 to 7, in particular within a range of 5 to 6. The predeterminable second increment can in particular amount to 1. The predeterminable second increment can be independent or dependent of the predeterminable first increment, in particular the predeterminable second increment can equal the predeterminable first increment.

A stimulation to be released (because the first counter is equal to or exceeds the predeterminable stimulating threshold) can be suppressed, if the second counter meets the third condition.

In an embodiment, the computer-readable program causes the processor to additionally perform the following steps when being executed on the processor: a) determining a second maximum value for a degree of fulfillment of the third condition by the second counter within the first time period since the start condition, when the first condition is met; b) storing the second maximum value in the at least one memory unit. Considering both the first and second counter as well as their maximum values of a degree of fulfillment provides a more consistent picture of the programmed therapy algorithms as multiple influences determining if the active implantable medical device is actually stimulating are factored in.

A second maximum value measuring a degree of fulfillment of the second condition by the second counter might help to keep track of a detection of factors or conditions that indicate that a therapy is not necessary, even if the first counter meets the first condition. The second maximum value is, in an embodiment, stored in the at least memory unit alongside the maximum value.

The second counter meeting the third condition may be in one example representative for a detection of supraventricular tachycardia (SVT) episodes. SVT episodes can be the result of physical activity like sports. The detection of SVT episodes therefore typically suppresses the stimulation with the stimulation unit as there is no need for a therapy.

The second counter can be independent of the first counter and can be incremented based on an evaluation of different aspects of the cardiac signal than the first counter, but the second counter can also comprise completely or partially all aspects that are considered when incrementing the first counter or even be dependent on the first counter.

Moreover, the third condition can be independent of the first condition but also be completely or partially dependent on the first condition. In one example, the third condition amounts to the first condition multiplied by 2 or by a value lying within a range of 1 to 6, in particular 2 to 6, in particular within a range of 3 to 4.

In an embodiment, the criticality increment is weighted with a weighting factor of higher than 1 depending on at least one weighting criterion, wherein the at least one weighting criterion is chosen from the following group: a number of detections the at least one detecting unit has already performed; programming parameters of the active implantable medical device; and an integrity value quantifying an integrity of electrodes of the at least one detecting unit.

The number of detections the at least one detecting unit has already performed thereby can assess the quality of a detection of the at least one detecting unit as older detecting units might be more prone to a loss of their integrity. The integrity value quantifies the integrity of electrodes of the at least one detecting unit directly by considering any known solutions to determine the integrity of electrodes of the at least one detecting unit. For example, a lead-failure detecting might be considered.

The programming parameters comprise, in an embodiment, the first condition, the third condition, the predeterminable first threshold. The programming parameters might also comprise the predeterminable first degree of fulfillment of the first condition, the predeterminable second degree of fulfillment of the first condition, the predeterminable third degree of fulfillment of the first condition, and/or the predeterminable absolute threshold.

Any of these weighting criteria can increase the criticality increment, if the weighting factor is higher than 1, or can decrease the criticality increment, if the weighting factor is below 1. Whether these weighting criteria should lead to an increase or decrease depends on what these weighting criteria are indicating. For example, if some or all programming parameters are set restrictively to minimize a chance of a stimulation, then the criticality increment should be increased also more restrictive upon fulfillment of the predeterminable criticality criterion .

In an embodiment, a time point upon which the maximum value or the local maximum values have occurred within the first time period is stored in the at least one memory unit. Saving additionally the time point upon which the maximum value or the local maximumvalues have occurred within the first time period has the effect that circadian factors and/or recurring factors that impact the cardiac rhythm might be recognized more easily.

For example, if multiple (local) maximum values occur roughly at similar times within their first time period, this can indicate that the patient is performing an activity that increased his or her cardiac rhythm, for example sports. Similar times comprise time differences between two time points of two maximum values of maximally an hour, in particular maximally 30 minutes, in particular maximally 15 minutes.

Another example for the multiple (local) maximum values occurring roughly at similar times within their first time period can be environmental influences that the patient is exposed to at similar times due to his or her everyday life. For instance, the patient might work in a store with a theft detection system based on magnetic fields and/or radio waves and passes through the theft detection system when entering and when leaving the store, which occurs roughly at similar times as his or her shift starts and ends.

In an embodiment, if it is determined that the multiple (local) maximum values occurring roughly at similar times within their first time period, the criticality increment is weighted with a weighting factor of higher than 1.

In an embodiment, the (local) maximum value is only stored in the at least one memory unit, if the maximum value is equal to or exceeds a predeterminable minimum threshold.

If the (local) maximum value is so small that the maximum value is below the predeterminable minimum threshold, then it can be assumed that the related cardiac signal is within a normal and healthy area. Not storing such a small (local) maximum value has the effect that space in the at least one memory unit can be saved for more relevant information.

In an embodiment, the predeterminable minimum threshold lies in a range of 3 % to 25 %, in particular in a range of 4 % to 12 %, in particular in a range of 8 % to 10 %, in particular in a range of 5 % to 7 %. The predeterminable minimum threshold amounts in particular to 5 %, in particular to 12 %. Non-sustained SVT episodes may trigger a storage after reaching at least five tachycardic intervals. So these five intervals in relation to the VT/VF detection criterion (most important range 20 to 40) may give a percentage value of 5 out of 40 = 12.5%, 5 out of 20 = 25%.

In an embodiment, all (local) maximum values within the predeterminable first time period that were determined to be critical are stored in the at least one memory unit. By storing all critical (local) maximum values, it is easier to evaluate a first time period with multiple critical quantitative values.

In an embodiment, the computer-readable program causes the processor to additionally perform the following steps when being executed on the processor: a) decrementing the first counter based on a further cardiac rhythm criterion, in particular based on atrial rhythm information, or when a fourth condition is met, wherein the fourth condition is met, when a further cardiac rhythm counter or an atrial rhythm counter meets a fifth condition.

In an embodiment, the first time period is a constant time period, wherein the start condition and the second condition are defined by a start and an end point of the first time period, respectively.

In an aspect, the present invention relates to a method for storing information detected by an active implantable medical device for stimulating a human or animal heart. This method comprises the steps as follows:
a) detecting, with at least one detecting unit, a cardiac signal comprising a plurality of cardiac rhythm intervals;
b) determining a length of each individual cardiac rhythm interval;
c) incrementing a first counter subsequent to meeting a start condition, if the length of an individual cardiac rhythm interval is below a predeterminable first threshold;
d) releasing a stimulation for the human or animal heart by at least one stimulation unit, when the first counter meets a first condition; and
e) determining a maximum value of a degree of fulfillment of the first condition by the first counter within a first time period since the start condition when a second condition is met;
f) storing the maximum value in at least one memory unit.

In an aspect, the present invention relates to a non-transitory computer program product that comprises a computer-readable program that causes a processor to perform the following steps, when executed on the processor:
a) detecting, with at least one detecting unit, a cardiac signal comprising a plurality of cardiac rhythm intervals;
b) determining a length of each individual cardiac rhythm interval;
c) incrementing a first counter subsequent to meeting a start condition, if the length of an individual cardiac rhythm interval is below a predeterminable first threshold;
d) releasing a stimulation for the human or animal heart by at least one stimulation unit, when the first counter meets a first condition; and
e) determining a maximum value of a degree of fulfillment of the first condition by the first counter within a first time period since the start condition when a second condition is met;
f) storing the maximum value in at least one memory unit.

All embodiments of the active implantable medical device can be combined in any desired way and can be transferred either individually or in any arbitrary combination to the described method and the described computer program product. Likewise, all embodiments of the described method can be combined in any desired way and can be transferred either individually or in any arbitrary combination to the active implantable medical device and to the computer program product. Finally, all embodiments described with respect to the computer program product can be combined in any desired way and can be transferred either individually or in any arbitrary combination to the described active implantable medical device or to the described method.

Further details of aspects of the present invention will be described in the following making reference to exemplary embodiments and accompanying Figures. In the Figures:
- Figure 1A: schematically illustrates an embodiment of an active implantable medical device;
- Figure 1B: schematically illustrates different component of the active implantable medical device of Figure 1A;
- Figure 2: illustrates a narrowly missed therapy incident;
- Figure 3: illustrates another narrowly missed therapy incident;
- Figure 4A: illustrates multiple narrowly missed therapy incidents;
- Figure 4B: illustrates the same multiple narrowly missed therapy incidents as Figure 4A and illustrates additional thresholds; and
- Figure 5: illustrates values of a criticality counter determined by the active implantable medical device of Figure 1A over multiple days.

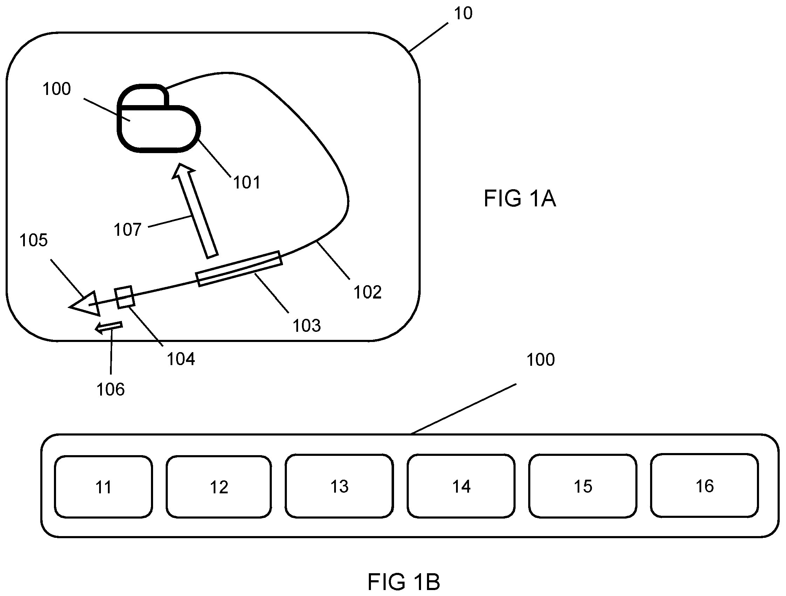

Figure 1A shows an implantable cardioverter-defibrillator (ICD) as an example of an active implantable medical device 10 for stimulating a human or animal heart. The active implantable medical device 10 comprises a main body 100 with a housing 101 and an electrode 102 that is implanted into or at body tissue of a patient. This electrode 102 receives stimulation pulses from a stimulation unit 14 arranged within the housing 101 of the main body 100 and is able to transfer cardiac signals from the patient's heart to a detecting unit 13 arranged within the housing 101 of the main body 100 (cf. Figure 1B for more details). This electrode 102 comprises at least three electrode poles 103, 104, 105, wherein a first electrode pole of the at least three electrode poles comprises a right ventricular (RV) tip electrode pole 105, a second electrode pole of the at least three electrode poles comprises a RV ring electrode pole 104, and a third electrode pole of the at least three electrode poles comprises a shock coil 103. A first vector 106 is spanned between the first electrode pole 105 and the second electrode pole 104. From this first vector 106 voltage signals can be derived and used for a detection by the detecting unit 13. A second vector 107 is spanned between the third electrode pole 103and the main body 100.

The example illustrated in Figure 1A shows a minimal configuration, but the present invention also works for dual chamber approaches or cardiac resynchronization therapy systems. In case the active implantable medical device comprises an integrated bipolar electrode lead, the RV ring electrode pole 104 is not present and the first vector 106 is spanned between the RV tip electrode pole 105 and the shock coil 103.

Figure 1B schematically illustrates individual components of the ICD device 10 of Figure 1A that are comprised within the housing 101. In this and in all following Figures, similar elements will be denoted with the same numeral reference. The housing 101 houses a detecting unit 13 (also referred to as sensing unit). The detecting unit 13 is arranged and designed to detect biological (physiologic) electrical signals of the patient via the electrode 102. The housing 101 further comprises a stimulation unit 14 that serves for stimulating the heart from which the detecting unit 13 detects cardiac signals. The electrode 102 forms part of the detecting unit 13 and of the stimulation unit 14. The detecting unit 13 and the stimulation unit 14 are operatively connected to a control unit 15 that, in turn, is operatively connected to a processor 11 that has access to a memory unit 12. The memory unit 12 serves for storing instructions for the processor 11 as well as data detected by the detecting unit 13. Additionally, the housing 101 comprises a data communication unit 16 that serves for data transfer to an external device.

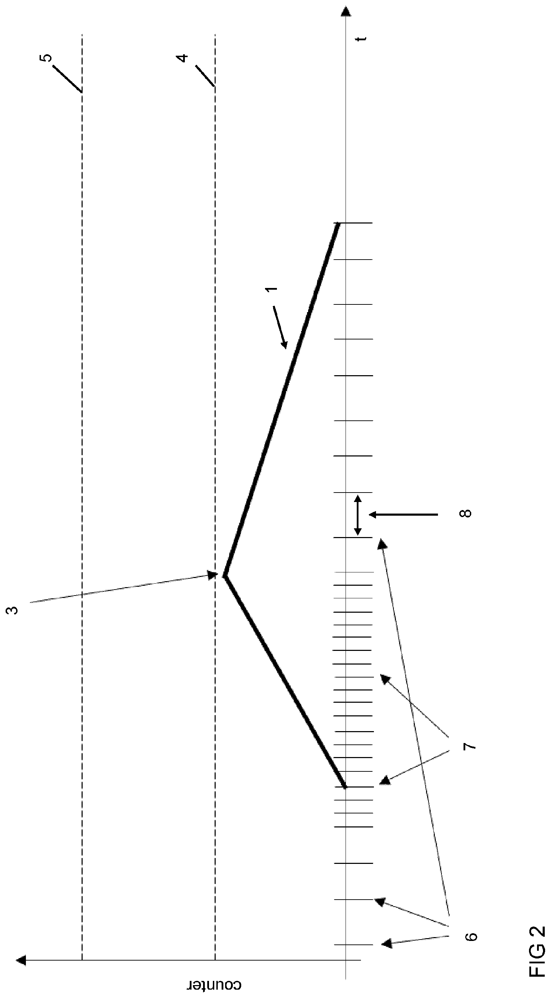

Figure 2 illustrates a narrowly missed therapy incident. For this purpose, Figure 2 shows a graph with an x-axis representing time and a y-axis representing counter values. The time extends over a few seconds or a few minutes, for example the time shown might lie in a range of 40 seconds to five minutes. Cardiac events detected by the active implantable medical device 10 of Figures 1A and 1B are shown as simple vertical lines on the x-axis, wherein a length (also referred to as distance) between two cardiac events is representing an RR interval 8. The shown RR intervals 8 comprise RR intervals indicative for tachycardia 7 and RR intervals 8 not indicative for tachycardia 6. RR intervals 8 that are indicative for tachycardia 7 have a shorter length than RR intervals 8 that are not indicative for tachycardia 6.

Furthermore, Figure 2 shows a curve representing values of the first counter 1 and two dotted lines representing a predeterminable stimulating threshold 4 as a first condition and a predeterminable non-stimulating threshold 5 as a third condition, wherein the predeterminable stimulating threshold 4 lies below the predeterminable non-stimulating threshold 5. The curve of the first counter 1 shows one peak below the predeterminable stimulating threshold 4. The increase of the first counter 1 is due to short RR intervals 8 and the decrease of the first counter 1 is due to longer RR intervals 8. Therefore, a faster heartbeat as indicated by the shorter RR intervals 8 normalized without an intervention in form of a stimulation. The one peak is a value of the first counter 1 that is fulfilling the predeterminable stimulating threshold 4 almost within the time shown. The degree of fulfillment of the predeterminable stimulating threshold 4 is determined in form of a quantitative value for the maximum value of the first counter 1 below the predeterminable stimulating threshold 4. The degree of fulfillment of the one peak is thereby relating to the quantitative value with the highest value within the time shown. Thus, the one peak is representative for the maximum value 3 for the time shown. If the first counter 1 would reach or exceed the predeterminable stimulating threshold 4, this would be representative for a detection of ventricular tachycardia (VT) and/or a ventricular fibrillation (VF) episodes. VT and/or VF episodes can be typically treated by a stimulation with the stimulation unit 14.

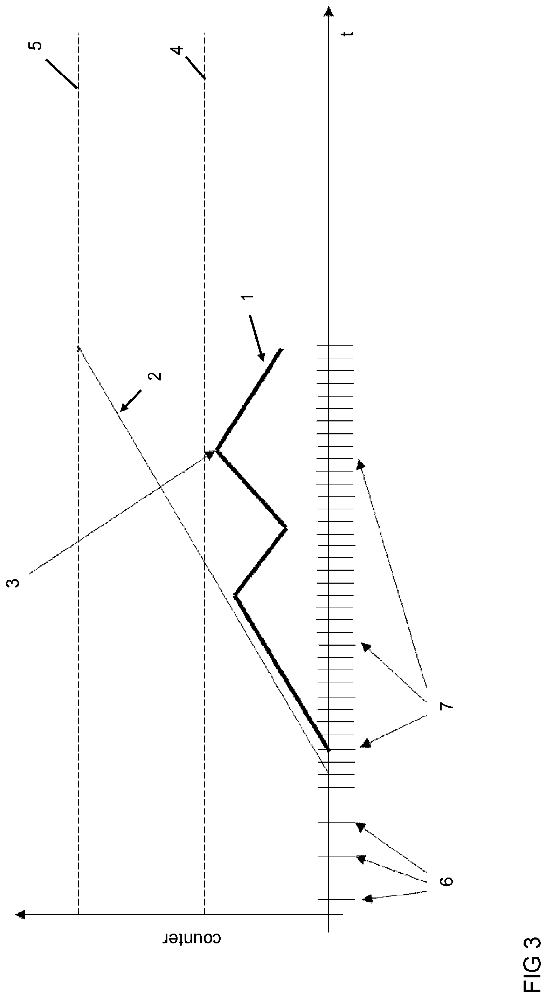

Figure 3 illustrates another narrowly missed therapy incident. Figure 3 shows a similar graph as Figure 2. However, Figure 3 shows a higher number of fast RR intervals 8. Furthermore, Figure 3 shows a different curve representing the first counter 1 and a curve representing a second counter 2. The different curve of the first counter 1 shows two peaks below the predeterminable stimulating threshold 4, wherein one peak is higher than the other one. The decrease of the first counter 1 is due to additional characteristics of the signals detected by the detecting unit 13 other than the RR intervals 8 that influence the first counter, for example atrial rhythm information. The higher peak is a value of the first counter 1 that is fulfilling the predeterminable stimulating threshold 4 almost within the time shown. The degree of fulfillment of the predeterminable stimulating threshold 4 is determined in form of the quantitative value for the maximum value of the first counter 1 below the predeterminable stimulating threshold 4. The degree of fulfillment of the higher peak is thereby relating to the quantitative value with the highest value. Thus, the higher peak is representative for the maximum value 3.

The curve of the second counter 2 shows a linear increase and the second counter 2 reaches the predeterminable non-stimulating threshold 5. If the second counter 2 is equal to the predeterminable non-stimulating threshold 5, the first time period is ended and the maximum value of a degree of fulfillment of the predeterminable stimulation threshold 4 is determined. The first counter is reset and is incremented again upon start of a new first time period after fulfillment of the start condition. A stimulation therapy is not applied during the just ended first time period. Reaching the predeterminable non-stimulating threshold is in this example representative for a detection of supraventricular tachycardia (SVT) episodes. SVT episodes can be the result of physical activity like sports, and the detection of SVT episodes therefore typically suppresses a stimulation with the stimulation unit 14 as there is no need for a therapy.

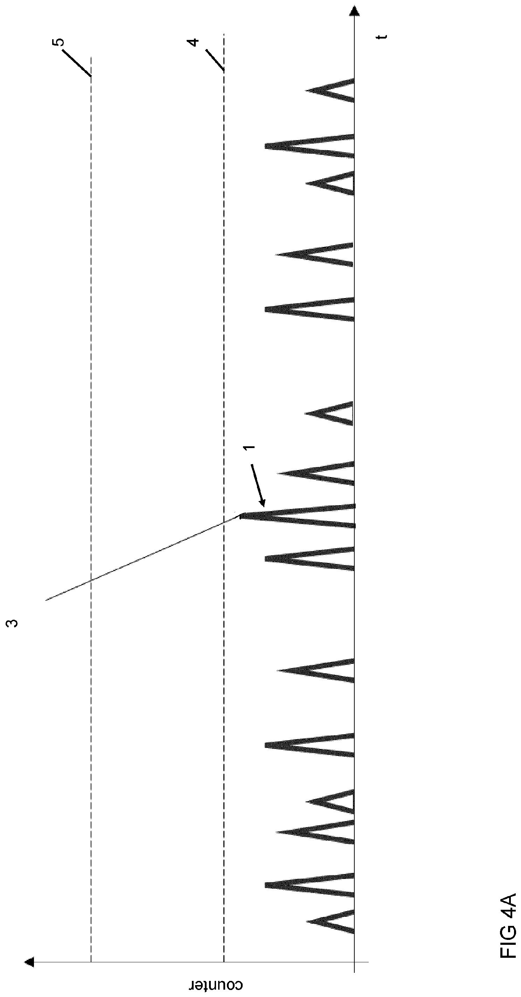

Figure 4A illustrates multiple narrowly missed therapy incidents. For this purpose, Figure 4A shows a graph the x-axis of which represents time and the y-axis of which represents counter values. Two dotted lines represent the predeterminable stimulating threshold 4 and the predeterminable non-stimulating threshold 5.

The time extends over multiple hours and can lie, e.g., in a range of two hours to 24 hours. Figure 4A shows multiple peaks, which all represent values of the first counter 1. All these peaks are below the predeterminable stimulating threshold. These peaks could be caused by oversensing of the detecting unit 13, physiological non-sustained tachycardia and/or sensing of electromagnetic fields by the active implantable medical device 10.

The highest peak in Figure 4A represents the maximum value 3 and can be seen as a representative of the predeterminable first time period as the maximum value is the closest the first counter 1 got within the first time period to fulfill the predeterminable stimulating threshold 4.

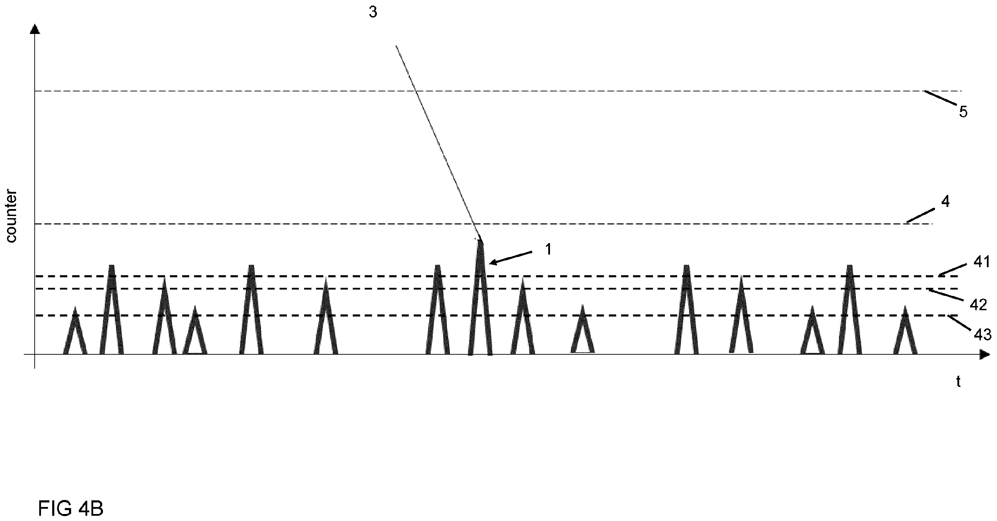

Figure 4B illustrates the same multiple narrowly missed therapy incidents as Figure 4A but shows six dotted lines representing the predeterminable stimulating threshold 4, a predeterminable first degree 41 of fulfillment of the predeterminable stimulating threshold 4, a predeterminable second degree 42 of fulfillment of the predeterminable stimulating threshold 4, a predeterminable third degree 43 of fulfillment of the predeterminable stimulating threshold 4, and the predeterminable non-stimulating threshold 5. The predeterminable third degree 43 of fulfillment of the predeterminable stimulating threshold 4 lies below the predeterminable second degree 42 of fulfillment of the predeterminable stimulating threshold 4, the predeterminable second degree 42 of fulfillment of the predeterminable stimulating threshold 4 lies below the predeterminable first degree 41 of fulfillment of the predeterminable stimulating threshold 4 and the predeterminable first degree 41 of fulfillment of the predeterminable stimulating threshold 4 lies below the predeterminable stimulating threshold 4.

As the maximum value 3 is greater than the predeterminable first degree 41 of fulfillment of the predeterminable stimulating threshold 4, a predeterminable criticality criterion is fulfilled. Thus, the maximum value 3 is considered critical. Some other peaks lie also above the predeterminable first degree 41 of fulfillment of the predeterminable stimulating threshold 4 and fulfill the predeterminable criticality criterion as well. Peaks that are below the predeterminable first degree 41 of fulfillment of the predeterminable stimulating threshold 4 but either above the predeterminable second degree 42 of fulfillment of the predeterminable stimulating threshold 4 or the predeterminable third degree 43 of fulfillment of the predeterminable stimulating threshold 4 fulfill the criticality criterion, if an additional condition is fulfilled. Typically, only the maximum value is stored in the memory unit 12, but it is also possible to store all quantitative values within the predeterminable first time period that are critical. Additionally or alternatively, the criticality increment could be weighted with a weighting factor if the predeterminable first time period comprises quantitative values besides the maximum value 3 that fulfill the predeterminable criticality criterion.

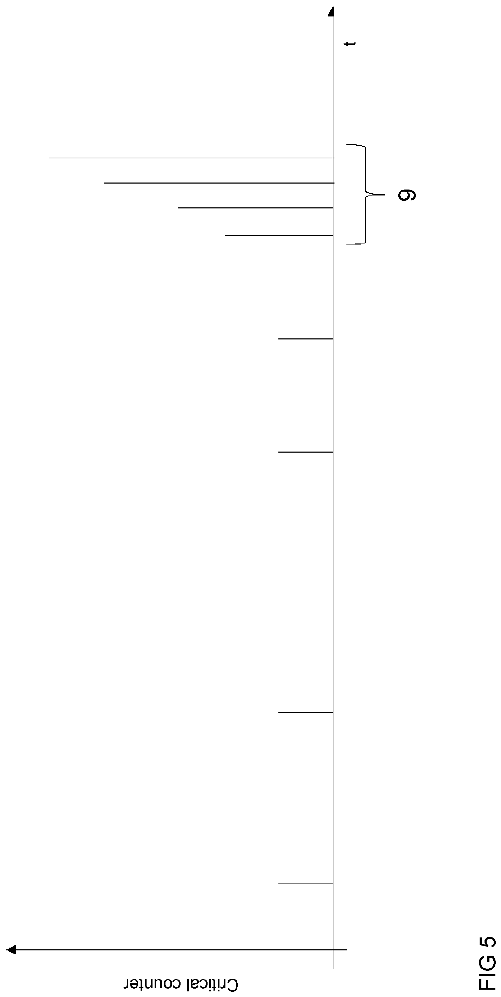

Figure 5 illustrates values of a criticality counter determined by the active implantable medical device of Figure 1A over multiple days. The graph of Figure 5 has an x-axis representing time and an y-axis representing criticality counter values. The time extends over days and can lie, e.g., in a range of 120 days to 240 days and amounts in particular to 180 days. As shown in Figure 5, some days comprise a criticality counter that amounts to zero, while for other days the criticality counter amounts to a small value, and for some critical days 9 that are consecutive to each other or are separated only by a few days the criticality counter amounts to a higher value and is increasing between these days. These critical days 9 are most likely the most relevant days as they can indicate a medical condition of a patient carrying the active implantable medical device 10, a loss of integrity of the electrode 102 or an environmental influence on the active implantable medical device 10. Each of these reasons might need to be addressed in order to avoid inadequate stimulations.

Figure 5 shows the benefit of determining a criticality of the maximum values within a predeterminable second time period and using a criticality counter to measure the criticality of all the maximum values within the predeterminable second time period because a trend as shown with the critical days 9 can be captured. The criticality is therefore a useful metric and can be the basis for further evaluation. The evaluation of the criticality counter for the days shown in Figure 5 leads to critical days 9 with an interesting trend. Here, further investigation of the underlying reasons for critical narrowly missed therapies is sensible. On the other hand, the criticality counter also indicates that most other days (due to the zero value or the small values of the criticality counter) are less relevant with respect to narrowly missed therapies. Here, a further investigation is not necessary. Thus, the criticality can help to separate the data in relevant data and less or not relevant data. This significantly facilitates data evaluation and helps in finding the cause of narrowly missed therapies. Consequently, this allows to take appropriate counter measures against physiologic or device-borne reasons for the detected narrowly missed therapies.

## Claims

1. Active implantable medical device (10) for stimulating a human or animal heart, comprising a processor (11), at least one memory unit (12), at least one detecting unit (13) configured to detect signals from the human or animal heart, and at least one stimulation unit (14) configured to stimulate the human or animal heart,
**characterized**
**in that** the at least one memory unit (12) comprises a computer-readable program that causes the processor (11) to perform the following steps when being executed on the processor (11):
a) detecting, with the at least one detecting unit (13), a cardiac signal comprising a plurality cardiac rhythm intervals;
b) determining a length of each individual cardiac rhythm interval;
c) incrementing a first counter (1) subsequent to meeting a start condition, if the length of an individual cardiac rhythm interval is below a predeterminable first threshold;
d) releasing a stimulation for the human or animal heart by the at least one stimulation unit (14), when the first counter (1) meets a first condition; and
e) determining a maximum value maximum, when a second condition is met;
f) storing the maximum value (3) in the at least one memory unit (12).

2. Active implantable medical device (10) according to claim 1, **characterized in that** the computer-readable program causes the processor (11) to additionally perform the following steps when being executed on the processor (11):
a) evaluating if the maximum value (3) is fulfilling a predeterminable criticality criterion;
b) if the predeterminable criticality criterion is fulfilled, triggering a signal generation and/or incrementing a criticality counter by a criticality increment and storing in the at least one memory unit (12) a value of the criticality counter assumed by the criticality counter at the end of a predeterminable second time period, the predeterminable second time period comprising at least the first time period.

3. Active implantable medical device (10) according to claim 2, **characterized in that** the predeterminable criticality criterion is fulfilled
- if the maximum value is equal to or exceeds a predeterminable first degree (41) of fulfillment of the first condition, and/or
- if the maximum value is equal to or exceeds a predeterminable second degree (42) of fulfillment of the first condition, wherein the predeterminable second degree (42) of fulfillment of the first condition is smaller than the predeterminable first degree (41) of fulfillment of the first condition, and if, at the same time, a majority of cardiac events detected within the first time period fulfills a first morphological criterion, and/or
- if the maximum value is equal to or exceeds a predeterminable absolute threshold.

4. Active implantable medical device (10) according to claim 3, **characterized in that** the predeterminable first degree (41) of fulfillment of the first condition lies within a range of 85 % to 95 %.

5. Active implantable medical device (10) according to claim 3 or 4, **characterized in that** the predeterminable second degree (42) of fulfillment of the first condition lies within a range of 65 % to 75 %.

6. Active implantable medical device (10) according to any one of claims 2 to 5, **characterized in that** the computer-readable program causes the processor (11) to determine local maximum values of a degree of fulfillment of the first condition by the first counter within the first time period and to store the local maximum values in the memory unit, wherein the predeterminable criticality criterion is fulfilled, if a predeterminable number of the local maximum values and the maximum value of a degree of fulfillment of the first condition within the first time period is equal to or exceeds a predeterminable third degree (43) of fulfillment of the first condition.

7. Active implantable medical device (10) according to claim 6, **characterized in that** the predeterminable third degree (43) of fulfillment of the first condition lies within a range of 45 % to 55 %.

8. Active implantable medical device (10) according to any of claims 2 to 7, **characterized in that** the criticality increment is weighted with a weighting factor of higher than 1 if the cardiac events detected within the first time period fulfills a second morphological criterion.

9. Active implantable medical device (10) according to any of claims 2 to 8, **characterized in that** the computer-readable program causes the processor (11) to additionally perform the following steps when being executed on the processor (11):
a) incrementing a second counter (2) together with the first counter (1) subsequent to meeting the start condition, if the length of an individual cardiac rhythm interval of the plurality of cardiac rhythm intervals is below the predeterminable first threshold, and wherein the second condition is met, when the second counter (2) meets a third condition, wherein the third condition is a supraventricular detection criterion.

10. Active implantable medical device (10) according to claim 9, **characterized in that** the computer-readable program causes the processor (11) to additionally perform the following steps when being executed on the processor (11):
a) determining a second maximum value for a degree of fulfillment of the third condition by the second counter (2) within the first time period since the start condition, when the first condition is met;
b) storing the second maximum value in the at least one memory unit (12).

11. Active implantable medical device (10) according to any of claims 1 to 10, **characterized in that** the start condition is fulfilled, when the length of each of at least two consecutive cardiac rhythm intervals is below the predeterminable first threshold, and/or **in that** the first condition is a ventricular tachycardia or ventricular fibrillation detection criterion.

12. Active implantable medical device (10) according to any of claims 1 to 11 **characterized in that** the computer-readable program causes the processor (11) to additionally perform the following steps when being executed on the processor (11):
a) decrementing the first counter (1) based on a further cardiac rhythm criterion, in particular based on atrial rhythm information or when a fourth condition is met, wherein the fourth condition is met, when a further cardiac rhythm counter or an atrial rhythm counter meets a fifth condition.

13. Active implantable medical device (10) according to any of claims 1 to 10 and 12 **characterized in that** the first time period is a constant time period, wherein the start condition and the second condition are defined by a start and an end point of the first time period, respectively.

14. Active implantable medical device (10) according to any of claims 2 to 13, **characterized in that** the criticality increment is weighted with a weighting factor of higher than 1 depending on at least one of the following weighting criteria:
- a number of detections the at least one detecting unit (13) has already performed;
- programming parameters of the active implantable medical device;
- an integrity value quantifying an integrity of electrodes (102) of the at least one detecting unit (13).

15. Active implantable medical device (10) according to any of the preceding claims, **characterized in that** a time point upon which the maximum value (3) has occurred within the first time period is stored in the at least one memory unit (12).

16. Method for storing information detected by an active implantable medical device (10) for stimulating a human or animal heart according to any of the preceding claims, **characterized by** the following steps:
a) detecting, with at least one detecting unit (13), a cardiac signal comprising a plurality of cardiac rhythm intervals;
b) determining a length of each individual cardiac rhythm interval;
c) incrementing a first counter (1) subsequent to meeting a start condition, if the length of an individual cardiac rhythm interval is below a predeterminable first threshold;
d) releasing a stimulation for the human or animal heart by at least one stimulation unit (14), when the first counter (1) meets a first condition; and
e) determining a maximum value of a degree of fulfillment of the first condition by the first counter (1) within a first time period since the start condition when a second condition is met;
f) storing the maximum value (3) in at least one memory unit (12).

17. Computer program product comprising a computer-readable program that causes a processor (11) to perform the following steps when executed on the processor (11):
a) detecting, with at least one detecting unit (13), a cardiac signal comprising a plurality of cardiac rhythm intervals;
b) determining a length of each individual cardiac rhythm interval;
c) incrementing a first counter (1) subsequent to meeting a start condition, if the length of an individual cardiac rhythm interval is below a predeterminable first threshold;
d) releasing a stimulation for the human or animal heart by at least one stimulation unit (14), when the first counter (1) meets a first condition; and
e) determining a maximum value of a degree of fulfillment of the first condition by the first counter (1) within a first time period since the start condition when a second condition is met;
f) storing the maximum value (3) in at least one memory unit (12).
